Europäisches Patentamt

European Patent Office

Office européen des brevets

(11) Numéro de publication: **0 323 782 B1**

## (12) FASCICULE DE BREVET EUROPEEN

(45) Date de publication de fascicule du brevet: **11.08.93**    (51) Int. Cl.5: **C12N 1/04**

(21) Numéro de dépôt: **88403305.1**

(22) Date de dépôt: **23.12.88**

(54) **Protoplastes stabilisés.**

(30) Priorité: **24.12.87 FR 8718186**

(43) Date de publication de la demande:
**12.07.89 Bulletin 89/28**

(45) Mention de la délivrance du brevet:
**11.08.93 Bulletin 93/32**

(84) Etats contractants désignés:
**AT BE CH DE ES FR GB GR IT LI LU NL SE**

(56) Documents cités:

CHEMICAL ABSTRACTS, vol. 100, no. 3, 16 janvier 1984, page 290, résumé no. 20315t, Columbus, Ohio, US; G. FONT DE VALDEZ et al.: "Comparative study of the efficiency of some additives in protecting lactic acid bacteria against freeze-drying"

CHEMICAL ABSTRACTS, vol. 104, no. 13, 31 mars 1986, page 405, résumé no. 106047b, Columbus, Ohio, US; T. HASHIBA et al.: "Technique for long-term preservation of protoplasts of fungi in liquid nitrogen"

(73) Titulaire: **ELF SANOFI**
**32-34, rue Marbeuf**
**F-75008 Paris(FR)**

(72) Inventeur: **Brousse, Michel**
**Val de la Saune Ste Foy d'Aigrefeuille**
**F-31570 Lanta(FR)**
Inventeur: **Jara, Patrick**
**27, rue des Iris Jaunes**
**F-31320 Castanet Tolosan(FR)**
Inventeur: **Deshayes, Christian**
**17, rue des Couteliers**
**F-31000 Toulouse(FR)**
Inventeur: **Lagarde, Gilles**
**9, Cours Debille**
**F-75011 Paris(FR)**

(74) Mandataire: **Gillard, Marie-Louise et al**
**Cabinet Beau de Loménie 158, rue de l'Université**
**F-75340 Paris Cédex 07 (FR)**

Rank Xerox (UK) Business Services
(3.10/3.6/3.3.1)

CHEMICAL ABSTRACTS, vol. 90, no. 7, 12 février 1979, pages 330-331, résumé no. 51596q, Columbus, Ohio, US; S.C. WIEST et al.: "Freeze-thaw injury to isolated spinach protoplasts and its simulation at above freezing temperatures"

CHEMICAL ABSTRACTS, vol. 70, no. 11, 17 mars 1969, page 89, résumé no. 44923c, Columbus, Ohio, US; G.A. SAMYGIN et al.: "Protective effect of solutions during drying of cells"

CHEMICAL ABSTRACTS, vol. 103, no. 24, 16 décembre 1985, page 311, résumé no. 200758k, Columbus, Ohio, US; S. HENRY-MICHELLAND et al.: "Study on the behavior of liposomes during freezing-thawing and lyophilization experiments. Effects of cryoprotectants"

CHEMICAL ABSTRACTS, vol. 105, no. 7, 18 août 1986, page 327, résumé no. 57428m, Columbus, Ohio, US; G. STRAUSS et al.: "The interaction of saccharides with lipid bilayer vesicles: stabilization during freeze-thawing and freeze-drying"

## Description

La présente invention concerne des compositions de protoplastes bactériens stabilisés, un procédé de préparation de ces compositions et un procédé de conservation des protoplastes bactériens.

Dans EP-A-0 246 163, on a décrit un procédé de traitement du lait en vue de l'obtention de fromages. Ce procédé consiste à ensemencer le lait à l'aide de protoplastes bactériens avant l'emprésurage de celui-ci.

Les protoplastes bactériens mis en oeuvre dans ce procédé sont préparés selon les techniques connues de l'art antérieur, qui consistent essentiellement à éliminer la paroi bactérienne. Selon une de ces techniques, on met on oeuvre du lysozyme pour la préparation de suspensions de protoplastes.

Ces suspensions de protoplastes sont à utiliser dans un délai très court après l'adjonction du lysozyme à la suspension bactérienne.

Pour un emploi différé, de telles suspensions doivent être stabilisées notamment par l'incorporation à la suspension de protoplastes d'un stabilisant osmotique tel que du saccharose ou du lactose, à la concentration de 0,5 M ou par reconstitution, à l'aide de ladite suspension de protoplastes et de lait écrémé en poudre, d'un lait dont la pression osmotique est voisine de celle du contenu intracellulaire des protoplastes.

Toutefois, de telles suspensions stabilisées ont une durée de conservation limitée.

On a maintenant trouvé de façon surprenante des compositions de protoplastes bactériens au sein desquelles les protoplastes sont stabilisés, c'est-à-dire ne subissent pas, au cours d'un temps de conservation prolongé, de dégradations qui, affectant leur membrane cellulaire, pourraient conduire à leur lyse.

Selon un premier aspect, l'invention concerne donc des compositions de protoplastes stabilisés caractérisées en ce qu'elles sont constituées d'une suspension, à l'état congelé ou à l'état lyophilisé, de protoplastes bactériens dans un milieu cryoprotecteur.

Le milieu cryoprotecteur mis en oeuvre selon l'invention est un milieu permettant de conserver l'intégrité des protoplastes lors de la congélation, de la lyophilisation et de la conservation ultérieure.

Ce milieu est avantageusement du lait écrémé ou une solution aqueuse comprenant un sucre.

Les sucres appropriés aux fins de l'invention sont des sucres dont les molécules ne pénètrent pas dans les protoplastes, par exemple à cause de leur masse moléculaire trop importante. Des sucres appréciés sont notamment les osides, en particulier les holosides ou les polysaccharides, tels que par exemple le saccharose, le maltose, le cellobiose, le mélibiose, le lactose, le tréhalose ou les maltodextrines.

La quantité de sucre dans le milieu cryoprotecteur est choisie de façon à avoir une pression osmotique de ce dernier proche de celle du contenu intracellulaire des protoplastes, laquelle dépend de la bactérie protoplastifiée. Par exemple, pour des protoplastes issus de Streptococcus thermophilus, on utilisera avantageusement des solutions aqueuses contenant des osides ayant une masse moléculaire d'environ 340 et dont l'osmolalité réelle en oside, mesurée sur un osmométre cryoscopique, est d'environ 650 milliosmoles.

De telles compositions peuvent être préparées à partir des souches bactériennes connues. Parmi celles-ci, les souches utiles pour une fermentation dans le domaine des industries agro-alimentaires constituent un matériel de choix. On apprécie spécialement parmi celles-ci les souches thermophiles et les souches mésophiles. Les souches de bactéries lactiques sont particulièrement bien adaptées.

Parmi ces souches lactiques, on peut classer notamment les genres Lactobacillus, Leuconostoc et Streptococcus ; on peut également citer les bactéries alcalinisantes du groupe corynéforme parmi lesquelles le genre Arthrobacter et l'espèce Brevibacterium linens jouent un rôle déterminant, ainsi que les microcoques (genre Micrococcus) ou les bactéries propioniques (genre Propionibacterium) dont l'action est bien connue pour les fromages présentant des ouvertures internes, tels que le gruyère de comté.

Selon un second aspect, l'invention concerne un procédé de préparation des compositions de protoplastes stabilisés qui comprend les étapes suivantes :

a) de formation de protoplastes à partir de bactéries ;
b) de congélation d'une suspension des protoplastes obtenus à l'étape a) dans un milieu cryoprotecteur ;
c) éventuellement, de lyophilisation de la suspension congelée ainsi obtenue.

La formation de protoplastes ou "protoplastisation" est réalisée, selon les techniques de l'art antérieur, par action d'enzymes capables de rompre la paroi bactérienne. On opère avantageusement en présence de lysozyme selon le mode opératoire décrit dans la demande de brevet EP-A-0 246 163 citée dans la présente description à titre de référence.

Selon un mode opératoire préféré, la protoplastisation est réalisée dans un milieu osmoprotecteur, c'est-à-dire un milieu permettant de conserver l'intégrité des protoplastes formés. On pense en effet que la pression osmotique de celui-ci joue un rôle important dans la stabilisation des protoplastes. On utilise avantageusement des solutions aqueuses de sucre, de préférence les mêmes solutions aqueuses que celles utilisées comme milieu cryoprotecteur. De telles solutions ont avantageusement une concentration molaire en sucre de 0,5 M. La suspension de protoplastes ainsi obtenue, éventuellement stabilisée par le milieu osmoprotecteur est ensuite soumise à une congélation dans un liquide cryoprotecteur tel que défini précédemment. De façon préférée, le milieu cryoprotecteur de l'étape b) comprend le milieu osmoprotecteur utilisé dans l'étape a).

La congélation doit nécessairement être rapide de manière à éviter la formation de cristaux de grande taille susceptibles d'altérer la membrane cellulaire des protoplastes.

Un traitement de congélation préféré consiste à placer, dans une enceinte dont la température est inférieure à -80°C, du milieu cryoprotecteur contenant $10^{10}$ à $10^{12}$ protoplastes par ml et réparti dans un ou plusieurs récipients de manière à constituer une couche de liquide de 0,5 à 2 cm d'épaisseur. Dans ces conditions, la composition congelée ainsi préparée acquiert une structure cristalline complète en 1 à 2 heures environ. Cette composition peut ensuite être éventuellement lyophilisée.

La lyophilisation doit être conduite selon les règles connues de l'homme de l'art en tenant compte de la nature fragile du matériel biologique ici considéré.

De préférence, la suspension congelée est ensuite lyophilisée pendant au moins 24 heures dans un lyophilisateur porté à -45°C ; en fin de lyophilisation, la composition résultante est avantageusement portée à 20°C pour éliminer l'humidité résiduelle.

Les compositions de protoplastes bactériens ainsi obtenues peuvent être utilisées dans le procédé selon le brevet EP-A-0 246 163.

Lorsqu'il s'agit de compositions congelées, il convient au préalable de placer les récipients les contenant à une température permettant leur décongélation. Lorsqu'il s'agit de compositions lyophilisées, il convient de les réhydrater avec de l'eau distillée.

Selon un autre aspect, l'invention concerne un procédé de conservation de protoplastes bactériens caractérisé en ce que a) les protoplastes sont introduits dans un liquide cryoprotecteur, b) La suspension résultante est soumise à une congélation et c) la composition congelée est stockée jusqu'à son utilisation. Un stockage à -80°C est particulièrement approprié.

Ce procédé de conservation peut être perfectionné en soumettant la composition congelée à une lyophilisation. La composition lyophilisée peut être stockée avantageusement à une température de + 4°C.

Les exemples ci-après illustrent de manière non limitative l'invention.

## EXEMPLE 1

## I - PREPARATION DE COMPOSITIONS LYOPHILISEES A BASE DE PROTOPLASTES

1) Souches

8 souches ont été mises en oeuvre. Ces souches sont toutes des souches connues auxquelles le public a accés ; elles peuvent notamment être obtenues auprès du **Centre National de Recherches Zootechniques (France) (CNRZ).**

Brevibacterium linens (CNRZ 221) (mésophile)

Leuconostoc cremoris (CNRZ 361) (mésophile)

Micrococcus sp. (CNRZ 468) (mésophile)

Propionibacterium freundereichii subsp. shermanii (CNRZ 82) ci-après Propionibacterium. (mésophile)

Streptococcus cremoris (CNRZ 106), ci-après S. cremoris (mésophile).

Streptococcus lactis (souche isolée à partir du mélange lyophilisé commercialisé par les laboratoires ROGER sous l'appellation "Ferments mésophiles pâtes molles"), ci-après S. Lactis (mésophile).

Streptococcus lactis diacetylactis (CNRZ 124), ci-après S. lactis diacetylactis (mésophile).

Streptococcus thermophilus (CNRZ 302), ci-après S. thermophilus (thermophile)

2) Milieux, solutions et réactifs utilisés pour la culture des souches, la protoplastisation ainsi que la congélation et la lyophilisation.

- Milieux de culture des souches :

Les souches ci-dessus ont été cultivées en phase de croissance dans l'un des milieux ci-après :

**MILIEU A**

(Milieu connu sous l'appellation M17 décrit par B. Terzaghi et al. dans Applied Microbiology, 23,6, 1975 : 807-813)

Composition

* <u>Partie a</u> :

| | |
|---|---|
| Peptone papaïnique de soja (Biokar, référence 116002) | 5 g |
| Peptone caséine viande (Biokar, référence 104008) | 5 g |
| Extrait de levure (Difco, référence 0127-05-3) | 2,5 g |
| Extrait de boeuf (Difco, référence 0126-01-08) | 5 g |
| Acide ascorbique | 0,5 g |
| Glycérophosphate disodique | 1 g |
| $MgSO_4$,7 $H_2O$ 1 M | 1 ml |
| Eau distillée | qsq 950 ml |

Après ajustement du pH à 7,2 avec NaOH 4N, la partie a est stérilisée par traitement thermique (120°C, 20 min).

* <u>Partie b</u> :

| | |
|---|---|
| Lactose | 10 g |
| Eau distillée | qsq 50 ml |

La partie b est stérilisée par filtration sur membrane de porosité 0,22 $\mu$m.
Le milieu A est obtenu en ajoutant la partie b à la partie a.

**MILIEU B**

| | |
|---|---|
| Bacto tryptic soy broth (Difco, référence 0370-01) | 40 g |
| Tampon MOPS acide 3-(n-morpholino) propane-sulfonique (Sigma, référence M 1254) | 2 g |
| Extrait de levure (Difco, référence 0127-05-3) | 5 g |
| Eau distillée | qsq 1 000 ml |

Après ajustement du pH à 7,2 avec de la soude 4N, le milieu est stérilisé par traitement thermique (120°C, 20 min).

**MILIEU C**

Composition:

```
D-glucose...................................................... 10    g
Extrait de boeuf (Difco, référence 0126-01-08)............. 10    g
Peptone trypsique caséine (Biokar, référence 104001)....... 10    g
Extrait de levure (Difco, référence 0127-05-3)............. 5     g
Acétate d'ammonium......................................... 5     g
Acétate de sodium.......................................... 2     g
Monolaurate de polyoxyéthylènesorbitane (Tween® 80)....... 1     g
MgSO₄,7H₂O................................................ 0,2   g
MnSO₄,4H₂O............................................... 0,05  g
Oligo-éléments en solution................................. 1     ml
        H₃BO₃.................................... 30  mg
        MnCl₂,4H₂O............................... 70  mg
        ZnCl₂................................... 200  mg
        Na₂MoO₄,2H₂O............................. 20  mg
        FeCl₃,6H₂O.............................. 50  mg


        CuSO₄,5H₂O............................. 200  mg
        Eau distillée....................qsp 500 ml
    Eau distillée.................................qsp 1000  ml
```

Le pH est ajusté à 6,7 avant stérilisation.
Le milieu est stérilisé par traitement thermique (120°C, 20 min).

**MILIEU D**

Composition :

- **Solution 1**

| | |
|---|---|
| D-glucose | 10 g |
| Na₂HPO₄,2H₂O | 2 g |
| KH₂PO₄ | 2 g |
| MgSO₄,7H₂O | 0,5 g |
| MnSO₄,4H₂O | 0,05 g |
| Eau distillée | qsq 900 ml |

Le pH est ajusté à 6,8 avant stérilisation.
La solution est stérilisée par filtration sur membrane de porosité 0,22 μm.

**- Solution 2**

Composition :

| Extrait de levure (Difco, référence 0127-05-3) | 10 g |
| Eau distillée | qsq 100 ml |

Le pH ajusté à 6,8 avant stérilisation.
La solution est stérilisée par traitement thermique (120°C, 20 min).
Le milieu D complet est obtenu en ajoutant la solution 2 à la solution 1.

- Solution de lysozyme : solution de lysozyme de blanc d'oeuf

Composition :

| Chlorhydrate de lysozyme des laboratoires G.ROGER (activité enzymatique 22.500 unités SHUGAR/mg) | 2,5 g |
| Eau distillée | qsq 100 ml |

Cette solution est stérilisée par filtration sur membrane de porosité 0,22 $\mu$m.

- Tampon de protoplastisation

Composition :

| Saccharose | 171 g |
| NaCl | 0,584 g |
| MgCl$_2$ | 1,02 g |
| Tampon tri (hydroxyméthyl) aminométhane-HCl 100 mM, pH 8 | qsq 1000 ml |

Le tampon est stérilisé par traitement thermique (120°C, 20 min).

- Milieu cryoprotecteur

Du lait écrémé est reconstitué par addition de 930 ml d'eau distillée à 100 g de poudre Régilait®. Il est stérilisé par traitement thermique (110°C, 20 min).

3) Mode opératoire

* Principe général :

Les souches sont mises en culture de manière à disposer pour chacune d'elles d'une suspension de travail.
L'obtention des protoplastes a pour point de départ ces suspensions de travail. Les suspensions de protoplastes obtenues sont soumises à une congélation puis à une lyophilisation.

7

a) Culture des souches

Les conditions de culture sont portées dans le tableau 1 ci-après.

TABLEAU 1

| SOUCHE | MILIEU | ATMOSPHERE | TEMPERATURE ($^\circ$C) | TEMPS DE PRECULTURE (h) | TEMPS DE CULTURE (h) |
|---|---|---|---|---|---|
| Brevibacterium linens | B | aérobiose | 30 | 5 | 12 |
| Leuconostoc cremoris | C | aérobiose | 30 | 5 | 18 |
| Micrococcus sp. | B | aérobiose | 30 | 5 | 8 |
| Propionibacterium | D | anaérobiose | 30 | - | 26 |
| S. cremoris | A | aérobiose | 30 | 5 | 10 |
| S. lactis | A | aérobiose | 30 | 5 | 8 |
| S. lactis diacetylactis | A | aérobiose | 30 | 5 | 10 |
| S. thermophilus | A | aérobiose | 37 | 5 | 16 |

Pour chaque souche à l'exception de celle de Propionibacterium, à partir d'une ampoule congelée à -80 $^\circ$C contenant environ 500 $\mu$l de suspension bactérienne, on inocule 10 ml de milieu de culture. Après incubation, cette préculture est transférée dans 500 ml de même milieu. La suspension obtenue est incubée sous agitation douce.

La souche de Propionibacterium est également mise en culture à partir d'une ampoule congelée. Elle n'est pas soumise à une préculture. Le milieu ensemencé directement est mis à incuber sans agitation.

Des suspensions de travail (ST) contenant environ $10^9$ bactéries dénombrées sous forme d'unités formant des colonies (UFC) sont recueillies. A ce stade, la culture est à la fin de la phase exponentielle de croissance.

b) Protoplastisation

1 l de suspension (ST) obtenue en a) est centrifugé (4825 g, 30 minutes).

Le surnageant (S1) est éliminé. Le culot bactérien (C1) est repris dans 300 ml d'eau physiologique. La suspension (SC1) obtenue est centrifugée (4825 g, 30 minutes). Le surnageant (S2) est éliminé. Le culot bactérien (C2) est repris dans 300 ml d'eau physiologique. La suspension (SC2) obtenue est centrifugée (4825 g, 30 minutes). Le surnageant (S3) est éliminé. Le culot bactérien (C3) est repris dans 130 ml de tampon de protoplastisation. La suspension (SC3) obtenue est alors homogénéisée par agitation.

Aux 130 ml de la suspension (SC3) qui contient environ $10^{12}$ UFC sont ajoutés 20 ml de la solution de lysozyme. Le mélange obtenu est mis à incuber à 44 $^\circ$C sous agitation douce pendant 4 heures pour la souche de Micrococcus sp. et 2 heures pour chacune des autres souches. La suspension obtenue (SP1) contient les protoplastes et des bactéries encore pourvues de leur paroi.

L'efficacité des opérations conduisant à l'obtention des protoplastes a été vérifiée par dénombrement des protoplastes contenus dans les suspensions (SP1) à l'aide d'une cellule de MALASSEZ après dilution dans une solution de saccharose 0,5 M et dénombrement des bactéries résiduelles après dilution dans une solution de SDS (dodécylsulfate de sodium) à 1 %. De plus, le nombre de bactéries résiduelles a été estimé après étalement de la suspension sur milieu gélosé. On a ainsi constaté que n'ont résisté au traitement décrit ci-dessus qu'une seule bactérie sur 10000 pour la suspension ST de S. lactis, qu'une seule bactérie sur 1000 pour les ST de Brevibacterium linens et qu'une seule bactérie sur 100 pour les suspensions ST de Micrococcus sp. et de Propionibacterium.

c) Congélation et lyophilisation

La suspension (SP1) obtenue en b est centrifugée (à 2830 g pendant 30 minutes, à +4 $^\circ$C). Le surnageant (S4) est éliminé. Le culot (C4) est repris dans 150 ml de la solution de saccharose 0,5 M. La suspension obtenue (SC4) est centrifugée (à 2830 g, pendant 30 minutes, à +4 $^\circ$C). Le surnageant (S5) est éliminé. Le culot (C5) est repris dans 12 ml de lait écrémé.

La suspension obtenue (SC5) est homogénéisée avec précaution par aspiration et refoulement dans une pipette de 25 ml, puis est répartie, en flacons de type pénicilline, de façon que la suspension ait une épaisseur de 5 mm ; des bouchons adaptés sont positionnés sur les flacons. Les flacons sont disposés sur

8

les plateaux d'un lyophilisateur.

Les plateaux sont portés dans un congélateur réglé à -80°C. Après un séjour d'au moins une heure dans cette enceinte, les plateaux sont introduits dans le lyophilisateur dont la cuve a été préalablement refroidie à -45°C.

La lyophilisation est réalisée en 24 heures en portant en fin de lyophilisation le produit à 20°C. La fermeture des flacons est assurée dans la cuve de sublimation par abaissement des plateaux.

## II - Caractéristiques des compositions lyophilisées

Les 8 compositions préparées en I s'avèrent contenir un nombre élevé de protoplastes par unité de volume, après réhydratation par addition d'eau physiologique sous un volume équivalent à celui que présentait la composition avant lyophilisation.

Les comptages de protoplastes ayant été effectués à l'aide d'une cellule de MALASSEZ, les résultats ci-après ont été obtenus, les rendements inscrits exprimant le pourcentage de protoplastes ayant résisté à la lyophilisation.

TABLEAU 2

|  | RENDEMENT DE LYOPHILISATION (%) |
|---|---|
| Brevibacterium linens | 41 |
| Leuconostoc cremoris | 24 |
| Micrococcus sp. | 50 |
| Propionibacterium | 33 |
| S. cremoris | 53 |
| S. lactis | 50 |
| S. lactis diacetylactis | 24 |
| S. thermophilus | 26 |

## III - UTILISATION DE COMPOSITIONS LYOPHILISEES A BASE DE PROTOPLASTES LYOPHILISES EN FABRICATION FROMAGERE

Deux essais ont été réalisés, l'un portant sur la fabrication d'un fromage à pâte molle stabilisée et à croûte mixte et morgée (1er essai), et l'autre portant sur la fabrication d'un fromage à pâte molle et à croûte lavée (2e essai). Ces essais ont été réalisés 48 heures après la préparation des protoplastes stabilisés.

Les fabrications ont été conduites de manière traditionnelle. Les protoplastes ont été ajoutés au lait en cuve de fabrication avant l'addition de présure.

Pour évaluer l'action des protoplastes, on a déterminé (1er essai) le degré d'affinage par mesure d'un paramètre physique. Des tests organoleptiques (2e essai) ont également été effectués.

1) Fabrication d'un fromage à pâte molle stabilisée et à croûte morgée mixte

a) Protocole de fabrication

La souche de Streptococcus lactis a été mise en oeuvre. Deux suspensions de protoplastes dans de l'eau physiologique, contenant l'une $10^{12}$ protoplastes et l'autre $10^{10}$ protoplastes, sont préparées à partir d'une composition lyophilisée telle qu'obtenue au paragraphe I.2.c.

L'essai a été partagé en 2 séries, différent l'une par rapport à l'autre par la quantité de protoplastes ajoutés, soit pour 100 litres de lait de fabrication non encore emprésuré :

* série 1 : $10^{10}$ protoplastes
* série 2 : $10^{12}$ protoplastes

9

**Description du fromage**

. Fromage maigre     : 25 % matière grasse/extrait sec
. Format                : unités de forme circulaire d'un poids moyen de 1,5 kg.

**Paramètres de fabrication**

Lait maturé à pH 6,4 avant addition de présure
Ensemencement en flore lactique avec ferments FLORA DANICA[R]
Addition de présure
Léger délactosage en cuve
Egouttage spontané jusqu'à pH 5,20
Salage en saumure pour obtenir un taux de sel compris entre 1,2 et 1,3 %.

**Paramètres d'affinage**

\* Nature des ensemencements microbiens :

Ensemencement dans le lait de microcoques et de ferments du rouge
Ensemencement en surface d'un cocktail microbien constitué de microcoques, de ferments du rouge et de spores de <u>Geotrichum candidum</u>

\* Conduite de l'affinage :

Maintien des fromages pendant 17 jours à 13-14°C
Emballage
Maintien des fromages à 8°C.

    Parallèlement aux séries 1 et 2, une fabrication témoin, pour laquelle il n'y a pas addition de protoplastes, a été réalisée.

    Des prélèvements d'echantillons sur les fromages des séries 1 et 2 et sur ceux de la fabrication témoin ont été affectués :

- 48 heures après sortie saumure,
- à l'emballage,
- 8 jours après l'emballage,

et 25 jours après l'emballage.

b) <u>Mesures effectuées</u>

    L'évolution du degré d'affinage a été suivie pendant 38 jours en effectuant, sur des extraits aqueux préparés à partir des échantillons prélevés, une mesure de leur point de congélation et le calcul de l'écart (dans le sens d'un abaissement) le séparant de celui de l'eau distillée.

1) <u>Méthode</u>

    La méthode mise en oeuvre est une adaptation de celle décrite par M. COURROYE (Revue Laitière Française, 462, 1987 : 53-54).

    Chaque échantillon est traité comme indiqué ci-après :

    30 à 40 g de matière prélevée par carottage vertical à mi-distance entre le bord extérieur et le centre de chaque pièce de fromage sont broyés à l'aide d'un mixeur à usage culinaire. 10 g du broyat sont remis en suspension dans 40 ml d'eau ultra-purifiée. Cette suspension est mise à incuber à 80°C pendant 20 minutes. L'extrait aqueux est prélevé. La pâte insoluble formée est lavée 2 fois avec 5 ml d'eau ultra-purifiée. Enfin, l'extrait aqueux et les eaux de lavage sont réunis et leur volume est complété, à l'aide d'eau ultra-purifiée, jusqu'à 50 ml.

    L'extrait global ainsi préparé est filtré sur papier WHATMAN® n° 42. Le filtrat est recueilli ; il est utilisé pour la détermination du degré d'affinage. Cette détermination s'effectue de manière indirecte par la mesure du point de congélation du filtrat puis le calcul de l'abaissement de celui-ci par rapport à celui de l'eau distillée. Plus le degré d'affinage est élevé et plus cet abaissement est important.

    L'appareil utilisé est un osmomètre automatique cryoscopique (Roebling®).

Cet appareil mesure, à partir d'une prise d'essai de 100 $\mu$l de filtrat, le point de congélation de celui-ci, détermine l'abaissement résultant en le comparant à celui de l'eau distillée et, convertissant la valeur obtenue, affiche directement l'osmolalité du filtrat.

Par souci de cohérence avec les données de M. COURROYE cité ci-dessus qui exprime le degré d'affinage en termes d'abaissement cryoscopique mesuré en °C, les valeurs d'osmolalité obtenues ont été converties en les reportant sur une courbe d'étalonnage construite à partir des données suivantes (tableau 3) :

TABLEAU 3

| SOLUTION DE NaCl CONTENANT Xg de NaCl PAR kg D'EAU | OSMOLALITE REELLE milliosmoles | ABAISSEMENT CRYOSCOPIQUE (°C) |
|---|---|---|
| X = 3,087 | 100 | 0,186 |
| X = 6,260 | 200 | 0,371 |
| X = 9,463 | 300 | 0,556 |
| X = 12,684 | 400 | 0,741 |
| X = 15,916 | 500 | 0,925 |
| X = 19,147 | 600 | 1,109 |
| X = 22,380 | 700 | 1,292 |

2) Résultats

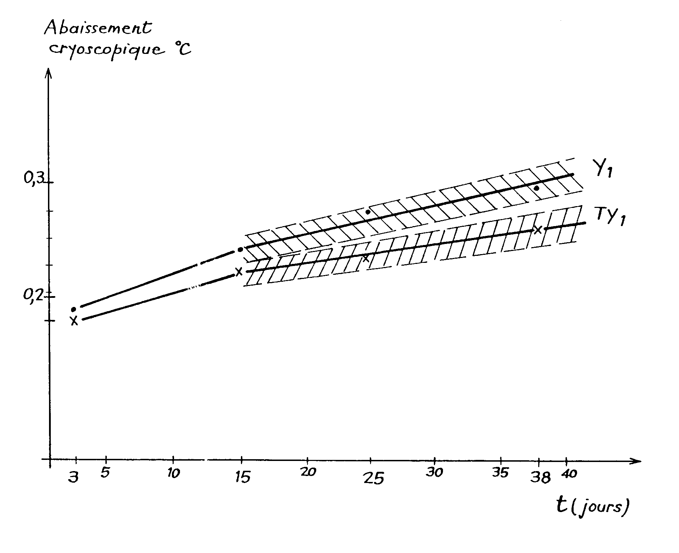

Les courbes portées sur la figure 1 rendent compte des mesures effectuées pour la série 1. La courbe Y1 correspond à la fabrication d'essai, la courbe TY1 correspond à une fabrication témoin réalisée dans des conditions identiques mais sans addition de protoplastes. On a vérifié par un test statistique que les écarts constatés entre les deux valeurs essai et témoin de chaque couple de mesures effectuées un même jour étaient significatifs.

On observe que l'affinage sur la fabrication d'essai est toujours à un stade plus avancé que celui de la fabrication témoin.

Un résultat identique a été observé avec la série 2.

Sur le tableau 4 ci-après, on a reporté les valeurs mesurées, pour cette série 2, le 38e jour d'affinage :

TABLEAU 4

| | ABAISSEMENT CRYOSCOPIQUE MESURE LE 38e JOUR | |
|---|---|---|
| | TEMOIN (°C) | ESSAI (°C) |
| SERIE N° 1 | 0,256 | 0,292 |
| SERIE N° 2 | 0,247 | 0,275 |

Il est ici constaté qu'il est possible en outre de contrôler le degré d'affinage en introduisant plus ou moins de protoplastes, l'affinage étant d'autant plus accéléré que la quantité de protoplastes ajoutée est importante.

2) Fabrication d'un fromage à pâte molle et à croûte lavée

a) Protocole de fabrication

4 séries d'expériences ont été réalisées. Pour chaque série, une suspension de protoplastes, préparée à partir d'une souche particulière, a servi à l'ensemencement de 100 litres de lait de fabrication non encore emprésuré (tableau 5).

11

TABLEAU 5

| SOUCHE | QUANTITE DE PROTOPLASTES POUR 100 LITRES DE LAIT |
|---|---|
| SERIE 1 Streptococcus lactis | $10^{13}$ |
| SERIE 2 Brevibacterium linens | $10^{12}$ |
| SERIE 3 Micrococcus sp. | $10^{12}$ |
| SERIE 4 Streptococcus thermophilus | $10^{12}$ |

**Description du fromage**

Pâte molle à croûte lavée,
45% de matière grasse/extrait sec,
Format : unités de forme circulaire et d'un poids moyen de 500 g.

**Paramètres de fabrication**

Lait prématuré 14 heures à 11° avec 0,1 % de ferments jusqu'à obtention d'un pH de 6,6
Maturation en cuve pendant 1 h 30 à 25°C avec 0,1 % de ferments,
Emprésurage à pH 6,45,
Décaillage puis moulage,
Egouttage en moule pendant 24 h,
Saumurage pendant 1 heure (taux de sel : 1,8 % dans le fromage).

**Paramètres d'affinage**

Ressuyage des fromages
Affinage des fromages à 13°C avec lavage deux fois par semaine avec une solution salée de Brevibacterium linens
     Les prélèvements d'echantillons ont été réalisés après :
- 30 jours d'affinage,
et 45 jours d'affinage.

b) Mesures effectuées

     Des tests organoleptiques ont été réalisés le trentième et le quarante-cinquième jour d'affinage :

1. Méthodes

     Les tests ont consisté pour un jury de 4 personnes à :
*    apprécier de manière visuelle le degré d'affinage
*    apprécier de manière gustative le degré d'affinage
*    apprécier globalement la présentation des fromages.

Les tableaux 6 et 7 rendent compte des résultats de ces tests.

**TABLEAU 6**

| Tests organoleptiques du trentième jour d'affinage | | | |
|---|---|---|---|
| Fabrication | appréciation globale de la présentation des fromages | appréciation visuelle du degré d'affinage | appréciation gustative du degré d'affinage |
| Témoin | Normale | Coeur dur important | Arôme non développé |
| Série 1 | Normale | Coeur légèrement dur | Arôme et goût |
| Série 2 | Normale | beaucoup moins étendu | développés et |
| Série 3 | Normale | que celui du témoin | caractéristiques |
| Série 4 | Normale | | |

13

## TABLEAU 7

| Fabrication | appréciation globale de la présenta-tion des fromages | appréciation visuelle du degré d'affinage | appréciation gustative du degré d'affinage |
|---|---|---|---|
| Témoin | Coulure sous la croûte importante | Coeur dur | Arôme plat |
| Série 1 | Normale | | Arôme et goût bien développés |
| Série 2 | Normale | Pâte souple Affinage sensible-ment identique à celui constaté le 30e jour | Tendance à l'amer-tume |
| Série 3 | Normale | | Arôme et goût bien développés |
| Série 4 | Normale | | Arôme et goût bien développés |

Tests organoleptiques du quarante-cinquième jour d'affinage

Préalablement à l'analyse des résultats, il doit être noté que l'obtention d'un arôme et d'un goût bien développés nécessite habituellement, pour une fabrication traditionnelle identique à celle ici mise en oeuvre pour le témoin, un affinage de deux à deux mois et demi et que le plus souvent certains défauts tels qu'une coulure sous la croûte sont effectivement constatés lorsque les fromages sont examinés le quarante-cinquième jour de l'affinage.

Les résultats présentés indiquent que l'addition de protoplastes a permis d'obtenir en 30 jours des fromages dans un état de maturation auquel on n'a pu parvenir sans protoplastes qu'au bout de 45 jours. L'accélération de l'affinage, ici mis en évidence, n'a pas été accompagnée d'une altération de leur valeur gustative ou de leur présentation. Il doit être d'autre part noté que d'une manière surprenante l'affinage de ces fromages d'essai s'est stabilisé : aucune évolution notable n'étant constatée entre le trentième et le quarante-cinquième jour d'affinage. Ce dernier résultat est remarquable ; ceci indique que de tels fromages pourraient être livrés après seulement 30 jours en cave d'affinage et être proposés à la vente sans évolution pendant une période prolongée.

Ces essais de fabrication de fromages à l'aide des compositions de protoplastes stabilisés de l'invention ont donc permis de confirmer l'intérêt de ces dernières dans l'industrie fromagère.

## EXEMPLE 2

**PREPARATION DE COMPOSITIONS LYOPHILISEES A BASE DE PROTOPLASTES ET CONTROLE DU TAUX DE LYSE DE CES DERNIERS**

1) Souches :

6 souches ont été mises en oeuvre.

| Microccus sp | CNRZ 468 (mésophile) |
|---|---|
| Streptococcus lactis | CNRZ 304 (mésophile) |
| Streptococcus cremoris | CNRZ 106 (mésophile) |
| Streptococcus diacetylactis | CNRZ 124 (mésophile) |
| Streptococcus thermophilus | CNRZ 302 (thermophile) |
| Streptococcus thermophilus | CNRZ 385 (thermophile) |

2) Milieux, solutions et réactifs :

- milieu de culture : milieu A de l'exemple 1
- solution de lysozyme : celle utilisée dans l'exemple 1
- tampon de protoplastisation : tampon PMNS

Composition

| Saccharose | 171 g/l |
|---|---|
| NaCl | 0,584 g/l |
| $MgCl_2$ | 1,02 g/l |
| Tampon Phosphate 0,1 M | qsp 1000 ml |

pH7, de composition suivante :
$NaH_2PO_4$ , $H_2O$ : 15,6 g/l et
$Na_2HPO_4$ , $12H_2O$ : 35,85 g/l.
Le tampon PMNS est filtré sur une membrane de seuil d'arrêt 0,22 $\mu$m.

**MILIEUX CRYOPROTECTEURS**

- Lait écrémé

Le lait écrémé est reconstitué par addition de 1000 ml d'eau distillée à 100 g de poudre de lait SKIM MILK commercialisée par la Société DIFCO. Il est stérilisé par traitement thermique (115°C, 20 min.)

- Solution de saccharose 0,5 M

| Saccharose (MERCK) | 171 g |
|---|---|
| Eau distillée   qsp | 1 litre |

Stérilisation par filtration sur membrane 0,22 $\mu$m.

15

- Solution de maltose 0,5 M

| Maltose monohydraté (PROLABO) | 180 g |
|---|---|
| Eau distillée   qsp | 1 litre |

Stérilisation par filtration sur membrane 0,22 $\mu$m.

- Solution de cellobiose 0,5 M

| D( + ) cellobiose (Fluka) | 171 g |
|---|---|
| Eau distillée   qsp | 1 litre |

Stérilisation par filtration sur 0,22 $\mu$m.

- Solution de mélibiose 0,5 M

| D( + ) mélibiose (Fluka) | 171 g |
|---|---|
| Eau distillée   qsp | 1 litre |

Stérilisation par filtration sur 0,22 $\mu$m.

- Solution de lactose 0,5 M

| Lactose monohydraté (Prolabo) | 180 g |
|---|---|
| Eau distillée   qsp | 1 litre |

Stérilisation par filtration sur 0,22 $\mu$m.

- Solution de tréhalose 0,5 M

| D( + ) Tréhalose (Sigma) | 189 g |
|---|---|
| Eau distillée   qsp | 1 litre |

Stérilisation par filtration sur 0,22 $\mu$m.

- Solution de maltodextrines

| Glucidex No. 6 (Roquette) | 200 g |
|---|---|
| Eau distillée   qsp | 1 litre |

Solution non stérilisée.

### 3) Mode opératoire

a) Culture des souches

Les cultures ont été effectuées à une température de 30°C dans le cas des souches mésophiles et à une température de 37°C dans le cas des souches thermophiles. Les cultures ont été effectuées en fioles de 2 l contenant 500 ml de milieu de culture (agitation 90-120 tours/min) pour les cinq premières souches et en fermenteurs, de 20 l contenant 15 l du milieu A pour la souche Streptococcus thermophilus CNRZ 385.

Les fermentations ont été arrêtées à la fin de la phase exponentielle de croissance.

b) protoplastisation

Les protoplastisations ont été effectuées selon des protocoles proches de ceux utilisés dans l'exemple 1.

L'efficacité de protoplastisation, vérifiée comme précédemment, est toujours supérieure à 99,9 %.

c) congélation et lyophilisation

Les échantillons à lyophiliser ont été placés dans des boîtes de Pétri de diamètre 90 mm, à raison de 10 ml par boîte puis congelés à une température de -80°C pendant au minimum 2 heures.

La lyophilisation a alors été effectuée pendant 48 heures dans un lyophilisateur, avec un chauffage à une température de 20°C pendant les deux dernières heures destiné à diminuer l'humidité résiduelle.

Les lyophilisats ont été conservés à une température de +4°C, dans des flacons bouchés mais non scellés.

### 4) Contrôle du taux de lyse des protoplastes par dosage LDH

Le taux de lyse des protoplastes avant la lyophilisation et à différentes durées après celle-ci a été déterminé par l'utilisation d'un marqueur enzymatique intracellulaire : la lactate déshydrogénase LDH, enzyme présente dans toutes les bactéries utilisées.

Ce taux de lyse T a été calculé sur des suspensions de protoplastes (reconstituées par addition d'eau distillée pour les compositions de protoplastes lyophilisées) par dosage de l'activité LDH totale de la suspension (activité obtenue après lyse totale des protoplastes sous l'effet d'un choc osmotique) et de celle du surnageant de cette suspension, selon la formule

$$T = 100 \times \frac{\text{activité LDH du surnageant}}{\text{activité LDH totale}}$$

17

Les principaux résultats obtenus sont rassemblés dans les tableaux 8 et 9 ci-après.

**TABLEAU 8**

Taux de lyse des protoplastes
de streptococcus thermophilus CNRZ 385
avec différents milieux cryoprotecteurs

| Taux de lyse<br>Milieu cryoprotecteur | Avant lyophi-lisation | 1 jour aprés lyophi-lisation | 15 jours aprés lyophi-lisation | 1 mois aprés lyophi-lisation |
|---|---|---|---|---|
| Solution de maltose 0,5 M (*) | 0,4 | 4 | 4 | 2 |
| Solution de cellobiose 0,5 M (*) | 0,4 | 5 | 6 | 5 |
| Solution de lactose 0,5 M (*) | 0,3 | 3 | 3 | 1 |
| Solution de mélibiose 0,5 M (*) | 0,7 | 6 | 4 | 5 |
| Solution de tréhalose 0,5 M (*) | 0,3 | 7 | 6 | 6 |
| Solution de maltodextrine 0,5 M | 1,3 | 4 | 5 | 4 |
| Solution de saccharose 0,5 M (*) | 0,6 | 7 | 5 | 6 |

(*) osmolalité réelle en oside d'environ 650 milliosmoles.

18

## TABLEAU 9

### Taux de lyse des protoplastes avec comme milieu cryoprotecteur
### le lait écrémé ou une solution de maltose 0,5 M
### pour différentes souches de bactéries lactiques

| Milieu cryopro- tecteur | Taux de lyse / souche | Avant lyophi- lisation | 1 jour aprés lyophi- lisation |
|---|---|---|---|
| LAIT ECREME | Micrococcus sp CNRZ 468 | 16 | 25 |
| | Streptococcus cremoris CNRZ 106 | 50 | 100 |
| | Streptococcus diacetylactis CNRZ 124 | <10 | <10 |
| | Streptococcus lactis CNRZ 304 | 100 | 100 |
| | Streptococcus thermophilus CNRZ 302 | 6 | 100 |
| SOLUTION DE MALTOSE 0,5 M | Micrococcus sp CNRZ 468 | 6 | 27 |
| | Streptococcus cremoris CNRZ 106 | 35 | 85 |
| | Streptococcus diacetylactis CNRZ 124 | >80 | 100 |
| | Streptococcus lactis CNRZ 304 | 25 | 100 |
| | Streptococcus thermophilus CNRZ 302 | 1 | <20 |

Le tableau 8 montre pour la souche Streptococcus thermophilus CNRZ 385 un taux de lyse faible avec les différents cryoprotecteurs exemplifiés.

Le tableau 9 montre que le lait écrémé est un bon cryoprotecteur pour la souche Streptococcus diacetylactis CNRZ 124 et que la solution de maltose 0,5 M est un bon cryoprotecteur pour la souche Streptococcus thermophilus CNRZ 302.

**EXEMPLE 3**

**ETUDE DE LA SIMPLIFICATION DU PROCEDE DE PREPARATION DES COMPOSITIONS DE PROTOPLASTES STABILISES**

On a inoculé 100 ml de milieu A avec environ 500 $\mu$l de suspension de bactéries de la souche Streptococcus thermophilus CNRZ 385. Après incubation pendant 6 heures, 10 ml de cette préculture ont ensuite été transférés dans 500 ml de milieu A. On a effectué la culture des bactéries à une température de 37°C pendant 6 heures sous agitation douce. La densité optique était à l'issue de cette période proche de 2.

On a alors prélevé le milieu de fermentation, on l'a centrifugé et repris le culot avec 500 ml de tampon PMNS (Cf. exemple 2). On a centrifugé à nouveau la suspension obtenue et on a repris le culot à l'aide de 50 ml de tampon PMNS. On a ainsi obtenu une suspension bactérienne qui a ensuite été transférée dans une cuve thermostatée à une température de 44°C, munie d'un système d'agitation magnétique. Après stabilisation de la température, on a ajouté à la suspension bactérienne une solution de lysozyme (celle utilisée dans les exemples 1 et 2) à la concentration de 1 g/l. On a laissé la protoplastisation se poursuivre pendant une heure quinze minutes. L'efficacité de protoplastisation, vérifiée comme précédemment, était alors d'environ 99,5 %.

On a prélevé dans le milieu de protoplastisation des échantillons pour congélation pendant 2 heures à une température de -80°C dans des flacons de verre. Certains de ceux-ci ont ensuite été lyophylisés pendant 48 heures, avec un chauffage à 20°C pendant les deux dernières heures pour diminuer l'humidité résiduelle. Les lyophilisats ont été conservés à une température de +4°C dans des flacons de verre.

Le taux de lyse des protopolastes à différentes étapes de la préparation des compositions de protoplastes stabilisés et au cours de leur conservation a été déterminé comme dans l'exemple 2 par dosage LDH. Les principaux résultats sont rassemblés dans le tableau 10 ci-après.

TABLEAU 10

| Taux de lyse des protoplastes de Streptococcus thermophilus CNRZ 385 avec une congélation et/ou une lyophilisation du milieu obtenu à l'issue de la protoplastisation | |
|---|---|
| | TAUX DE LYSE |
| à l'issue de la protoplastisation | 0,45 |
| à l'issue de la congélation | 1,6 |
| 15 jours après la congélation | 2,6 |
| 30 jours après la congélation | 1,7 |
| à l'issue de la lyophilisation | 12,3 |
| 15 jours après la lyophilisation | 9,2 |
| 30 jours après la lyophilisation | 13,5 |

Le tableau 10 montre un taux de lyse faible pour les protoplastes de la souche CNRZ 385. Il apparaît donc que le milieu entourant les protoplastes, obtenu à l'issue de la protoplastisation, lequel comprend une solution aqueuse de saccharose 0,5 M, est un bon cryoprotecteur pour cette souche. Les autres constituants de ce milieu ne gênent donc pas de façon importante l'effet cryoprotecteur de la solution de saccharose 0,5 M constaté précedemment (Cf. exemple 2).

La composition de protoplastes stabilisés ainsi obtenue après lyophilisation, présente toutefois l'inconvénient d'être hygroscopique et difficile à rendre pulvérulente. Ce défaut peut être supprimé grâce à l'ajout de silice hydratée, le Tixosil 38 A (Rhone Poulenc) de granulométrie 70 $\mu$m, à la suspension obtenue à l'issue de la protoplastisation.

Il est donc possible de simplifier considérablement le procédé de préparation des compositions de protoplastes stabilisés par l'utilisation comme cryoprotecteur du milieu entourant les protoplastes obtenu à l'issue de la protoplastisation.

**Revendications**

1. Composition de protoplastes stabilisés, caractérisée en ce qu'elle est constituée d'une suspension, à l'état congelé ou à l'état lyophilisé, de protoplastes bactériens dans un milieu cryoprotecteur.

**2.** Composition selon la revendication 1, caractérisée en ce que le milieu cryoprotecteur est une solution aqueuse comprenant un sucre.

**3.** Composition selon la revendication 2, caractérisée en ce que le sucre est un oside.

**4.** Composition selon la revendication 2, caractérisée en ce que le sucre est un polysaccharide.

**5.** Composition selon la revendication 1, caractérisée en ce que le milieu cryoprotecteur est du lait écrémé.

**6.** Composition selon l'une des revendications 1 à 5, caractérisée en ce que les protoplastes sont issus d'une souche bactérienne utile pour une fermentation dans le domaine des industries agroalimentaires.

**7.** Composition selon la revendication 6, caractérisée en ce que la souche bactérienne est une souche de bactéries lactiques.

**8.** Procédé de préparation d'une composition selon l'une des revendications 1 à 7, caractérisé en ce qu'il comporte les étapes suivantes :
a) protoplastisation de bactéries ;
b) congélation d'une suspension des protoplastes obtenus à l'étape a) dans un milieu cryoprotecteur;
c) éventuellement, de lyophilisation de la suspension congelée ainsi obtenue.

**9.** Procédé selon la revendication 8, caractérisé en ce que l'étape a) est réalisée dans un milieu osmoprotecteur.

**10.** Procédé selon la revendication 9, caractérisé en ce que le milieu cryoprotecteur de l'étape b) comprend le milieu osmoprotecteur de l'étape a).

**11.** Procédé de conservation de protoplastes bactériens caractérisé en ce qu'il comprend les étapes suivantes :
a) introduction des protoplastes dans un milieu cryoprotecteur ;
b) congélation de la suspension résultante ;
c) stockage de la suspension congelée jusqu'à son utilisation.

**12.** Procédé selon la revendication 11, caractérisé en ce que à l'issue de l'étape b) la suspension congelée est soumise à une lyophilisation.

**Claims**

**1.** Composition of stabilized protoplasts, characterized in that it consists of a suspension of bacterial protoplasts, in the frozen or lyophilized state, in a cryoprotective medium.

**2.** Composition according to claim 1, characterized in that the cryoprotective medium is an aqueous solution containing a sugar.

**3.** Composition according to claim 2, characterized in that the sugar is an oside.

**4.** Composition according to claim 2, characterized in that the sugar is a polysaccharide.

**5.** Composition according to claim 1, characterized in that the cryoprotective medium is skimmed milk.

**6.** Composition according to one of claims 1 to 5, characterized in that the protoplasts are derived from a bacterial strain used for fermentation purposes in the agri-foodstuffs industries.

**7.** Composition according to claim 6, characterized in that the bacterial strain is a strain of lactic bacteria.

EP 0 323 782 B1

**8.** Process for the preparation of a composition according to one of claims 1 to 7, characterized in that it consists in the following steps :
a) protoplast formation from bacteria
b) freezing of the suspension of protoplasts obtained in step a) in a cryoprotective medium.
c) optionally, lyophilization of the frozen suspension thus obtained.

**9.** Process according to claim 8, characterized in that step a) is carried out in an osmoprotective medium.

**10.** Process according to one of claims 8 and 9, characterized in that the cryoprotective medium of step b) comprises the osmoprotective medium of step a).

**11.** Process for the conservation of bacterial protoplasts, characterized in that it comprises the following steps :
a) introduction of the protoplasts into a cryoprotective medium,
b) freezing of the resulting suspension,
c) storage of the frozen suspension until it is used.

**12.** Process according to claim 11, characterized in that at the end of step b) the frozen suspension is lyophilized.

**Patentansprüche**

**1.** Stabilisierte Protoplasten enthaltende Zusammensetzung, dadurch gekennzeichnet, daß sie aus einer in gefrorenem oder lyophilisiertem Zustand vorliegenden Suspension von bakteriellen Protoplasten in einem Tieftemperatur-Schutzmedium besteht.

**2.** Zusammensetzung nach Anspruch 1, dadurch gekennzeichnet, daß es sich beim Tieftemperatur-Schutzmedium um eine wäßrige Lösung mit einem Gehalt an einem Zucker handelt.

**3.** Zusammensetzung nach Anspruch 2, dadurch gekennzeichnet, daß es sich beim Zucker um ein Osid handelt.

**4.** Zusammensetzung nach Anspruch 2, dadurch gekennzeichnet, daß es sich beim Zucker um ein Polysaccharid handelt.

**5.** Zusammensetzung nach Anspruch 1, dadurch gekennzeichnet, daß es sich beim Tieftemperatur-Schutzmedium um entrahmte Milch handelt.

**6.** Zusammensetzung nach einem der Ansprüche 1 bis 5, dadurch gekennzeichnet, daß sich die Protoplasten von einem Bakterienstamm ableiten, der sich für eine Fermentation im Bereich der Agrarnahrungsmittel-Industrie eignet.

**7.** Zusammensetzung nach Anspruch 6, dadurch gekennzeichnet, daß es sich beim Bakterienstamm um einen Stamm von Milchsäurebakterien handelt.

**8.** Verfahren zur Herstellung einer Zusammensetzung nach einem der Ansprüche 1 bis 7, dadurch gekennzeichnet, daß es folgende Stufen umfaßt:
(a) Bildung von Protoplasten aus den Bakterien;
(b) Einfrieren einer Suspension der in Stufe (a) erhaltenen Protoplasten in einem Tieftemperatur-Schutzmedium; und
(c) gegebenenfalls Lyophilisieren der auf diese Weise erhaltenen eingefrorenen Suspension.

**9.** Verfahren nach Anspruch 8, dadurch gekennzeichnet, daß die Stufe (a) in einem Osmose-Schutzmedium durchgeführt wird.

**10.** Verfahren nach einem der Ansprüche 8 und 9, dadurch gekennzeichnet, daß das Tieftemperatur-Schutzmedium in Stufe (b) das Osmose-Schutzmedium von Stufe (a) umfaßt.

22

EP 0 323 782 B1

**11.** Verfahren zur Konservierung von bakteriellen Protoplasten, dadurch gekennzeichnet, daß es folgende Stufen umfaßt:

(a) Einführen der Protoplasten in ein Tieftemperatur-Schutzmedium;

(b) Einfrieren der erhaltenen Suspension; und

(c) Aufbewahren der eingefrorenen Suspension bis zur Verwendung.

**12.** Verfahren nach Anspruch 11, dadurch gekennzeichnet, daß nach Beendigung der Stufe (b) die eingefrorene Suspension einer Lyophilisation unterzogen wird.

23